(19) Europäisches Patentamt
European Patent Office
Office européen des brevets

(11) Publication number: 0 151 351
B1

# (12) EUROPEAN PATENT SPECIFICATION

(45) Date of publication of patent specification: 03.08.88

(51) Int. Cl.[4]: C 07 C 5/27, C 07 C 15/08 // B01J29/28

(21) Application number: 84308934.3

(22) Date of filing: 20.12.84

## (54) Process for isomerizing alkyl aromatic hydrocarbons.

(30) Priority: 11.01.84 US 569830
11.01.84 US 569862
11.01.84 US 569827
11.01.84 US 569828

(43) Date of publication of application:
14.08.85 Bulletin 85/33

(45) Publication of the grant of the patent:
03.08.88 Bulletin 88/31

(84) Designated Contracting States:
BE DE FR GB IT NL

(58) References cited:
EP-A-0 102 716
GB-A-1 583 707
US-A-4 104 151

(73) Proprietor: MOBIL OIL CORPORATION
150 East 42nd Street
New York New York 10017 (US)

(72) Inventor: Chu, Yung-Feng
5 Lakeview Drive
Cherry Hill, New Jersey 08003 (US)
Inventor: Vartuli, James Clarke
320 Ponds Edge Road
West Chester, Pennsylvania 19380 (US)

(74) Representative: Cooper, John Anthony et al
Mobil Court 3 Clements Inn
London WC2A 2EB (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 151 351 B1

**Description**

This invention relates to a process for the isomerization of alkyl aromatics and, in particular, for the isomerization of ethylbenzene to xylenes.

Isomerization of alkyl aromatics, particularly xylenes, has become important commercially. In processes for the production of xylenes, the ortho and paraxylenes are the preferred products. Paraxylene is principally used in preparation of polyesters, while orthoxylene's main end use is in preparation of phthalic anhydride. Metaxylene has fewer important end uses and thus may be converted to the para and ortho forms, which have greater commercial value.

Ethylbenzene is difficult to separate from the xylenes since their boiling points are very close. Accordingly, ethylbenzene is normally present within the mixture of xylenes prepared by extraction or distillation from a hydrocarbon stream. Generally there are two approaches to the problem of processing the ethylbenzene in a mixed xylene stream. Because ethylbenzene is not easily isomerized one approach has been to destroy the ethylbenzene through disproportionation, hydrodealkylation or the like to yield lighter and heavier compounds which can be separated by distillation from the $C_8$ compounds. One drawback to this approach has been the accompanying loss of significant quantities of potential xylenes in this reaction.

It is also known in the prior art to isomerize ethylbenzene to form xylenes in the presence of hydrogen and a hydrogenation-dehydrogenation catalyst, preferably platinum on alumina. For example, U.S. Patent 2,976,332 discloses a catalyst comprising a combination of platinum on alumina with amorphous silica/alumina for isomerizing ethylbenzene and xylenes with a minimum of side reactions which reduce the yield selectivity and contribute to catalyst ageing. Other combination catalysts which are capable of isomerizing xylenes and ethylbenzenes to approach an equilibrium distribution of isomers are disclosed in U.S. Patent 3,409,686 in which alumina gel is mixed with particles of hydrogen mordenite to form a mixed base which is subsequently dried and impregnated with a platinum solution. In U.S. Patent 3,767,721 platinum on alumina in a fine powdered form is combined with powdered mordenite, but the hydrogen mordenite in such a combination is overly active and promotes destructive reactions which are undesirable. U.S. Patent 4,128,591 discloses a catalyst and process for producing a near equilibrium mixture of xylene from a feedstream comprising ethylbenzene and mixed xylenes, wherein the catalyst combines a platinum containing hydrogenation/dehydrogenation component on an alumina support with a form of hydrogen mordenite.

While some of the prior art catalysts have been successful in isomerizing ethylbenzene in the presence of xylenes, further improvement has been desired in order to achieve a more selective catalyst which can be operated to approach chemical equilibrium in the isomerization of xylenes and ethylbenzene while at the same time avoiding the destructive reactions which can result in a loss of $C_8$ aromatics. GB—A—1,583,707 discloses the use of a variety of metal-containing zeolite catalysts for isomerisation of ethylbenzene. Prior filed EP—A—102,716 discloses the use of a ZSM-22 for isomerisation of a xylene feed which may contain ethylbenzene.

According to the invention, there is provided a process for isomerizing ethylbenzene selectively to provide para xylene comprising passing ethyl benzene and hydrogen under isomerizing conditions over a catalyst comprising:

(a) ZSM-22 or ZSM-23 zeolite, and

(b) a hydrogenation/dehydrogenation metal, wherein the hydrogenation/dehydrogenation metal is incorporated in the catalyst after any steaming of the zeolite.

ZSM-22 is a highly siliceous zeolite which can be prepared from a reaction mixture containing a source of silica, $Q_2O$, an alkali metal oxide, e.g., sodium, potassium or cesium, water, and alumina, and having a composition, in terms of mole ratios of oxides, falling within the following ratios:

| Reactants | | Broad | Preferred |
|---|---|---|---|
| $SiO_2/Al_2O_3$ | = | 20 to ∞ | 30 to 1000 |
| $M_{2/n}O/(Q_2O+M_{2/n}O)$ | = | 0 to 0.95 | 0.1 to 0.8 |

wherein $Q_2O$ is the oxide form of an organic compound of an element of Group 5-B of the Periodic Table, e.g., N, P, preferably N, containing at least one alkyl or aryl group having at least 2 carbon atoms, and M is an alkali or alkaline earth metal of valence n, and maintaining the mixture at crystallization temperature until crystals of the ZSM-22 zeolite are formed. Thereafter, the crystals are separated from the liquid by any conventional means, washed and recovered.

Crystallization can be carried out under either static or stirred conditions in a reactor vessel, e.g., a polypropylene jar, teflon lined or stainless steel autoclaves, at 80°C (176°F) to 210°C (410°F) for 6 hours to 150 days. Thereafter, the crystals are separated from the liquid and recovered. The composition can be prepared utilizing materials which supply the appropriate oxide. Such materials include aluminates, silicates, silica hydrosol, silica gel, silicic acid, sodium, potassium or cesium hydroxide.

The organic compound contains an element of Group 5-B, such as nitrogen or phosphorus, preferably nitrogen. The preferred compounds are generally expressed by the following formula:

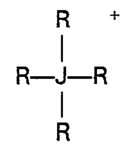

or $R_4J^+$

wherein J is an element of Group 5-B of the Periodic Table, e.g. N or P, preferably N, and each R is an alkyl or aryl group having at least two (2) carbon atoms or hydrogen. Suitable organic compounds are dialkylammonium compounds wherein each of the alkyl groups is the same or different and each alkyl group has two (2) to eight (8) carbon atoms, e.g., ethyl, propyl, butyl, pentyl, hexyl, heptyl or octyl. The reaction mixture can be prepared either batchwise or continuously. Crystal size and crystallization time of the new crystalline material will vary with the nature of the reaction mixture employed and the crystallization conditions.

The highly siliceous ZSM-22 zeolite comprises crystalline, three-dimensional continuous framework silicon-containing structures or crystals which result when all the oxygen atoms in the tetrahedra are mutually shared between tetrahedral atoms of silicon or aluminum, and which can exist with a network of mostly $SiO_2$, i.e., exclusive of any intracrystalline cations. Similar crystals form building blocks of materials, such as quartz, cristobalite and a long list of zeolite structures such as ZSM-5, ZSM-11, ZSM-12, ZSM-23, ZSM-35, ZSM-38, ZSM-48 (described in European Patent Number 0,015,132), mordenite and perhaps even faujasite. Not all zeolite structures are known to exist at this time in predominantly $SiO_2$—containing compositions—so the above class of materials does not presently include some zeolite, such as zeolite A.

The ZSM-22 zeolite also may contain a relatively minor amount of $Al_2O_3$ and therefore can produce a product with a $SiO_2$ to $Al_2O_3$ ratio of about 20 to about infinity. In the as-synthesized form, the ZSM-22 has a calculated composition, in terms of moles of oxides, after dehydration, per 100 moles of silica, as follows:

$$(x)Q_2O:(y)M_{2/n}O:(z)L_2O_3:100SiO_2$$

wherein $Q_2O$ is the oxide form of an organic compound containing an element of Group 5-B (as defined in the Table of the Elements—National Bureau of Standards, Fischer Scientific Co. Catalog No. 5-702-10) e.g., N or P, preferably N, containing at least one alkyl or aryl group having at least 2 carbon atoms, M is an alkali metal or an alkaline earth metal having a valence n, and wherein x=0.01—2.0, y=0—2.0, z=0—5, and L=Al.

ZSM-22 can further be identified by its sorptive characteristics and its X-ray diffraction pattern. The original cations of the as-synthesized ZSM-22 may be replaced at least in part by other ions using conventional ion exchange techniques. It may be necessary to precalcine the ZSM-22 zeolite crystals prior to ion exchange. The replacing ions introduced to replace the original alkali, alkaline earth and/or organic cations may be any that are desired so long as they can pass through the channels within the zeolite crystals. Desired replacing ions are those of hydrogen, rare earth metals, metals of Groups IB, IIA, IIB, IIIA, IIIB, IVA, IVB, VIB and VIII of the Periodic Table. Among the metals, those particularly preferred are rare earth metals, manganese, zinc and those of Group VIII of the Periodic Table.

ZSM-22 zeolite described herein has a definite X-ray diffraction pattern, set forth in Table I, which distinguishes it from other crystalline materials.

TABLE I
Most significant lines of ZSM-22

| Interplanar d-spacings (A) | Relative intensity |
|---|---|
| 10.9±0.2 | M—VS |
| 8.7 ±0.16 | W |
| 6.94±0.10 | W—M |
| 5.40±0.08 | W |
| 4.58±0.07 | W |
| 4.36±0.07 | VS |
| 3.68±0.05 | VS |
| 3.62±0.05 | S—VS |
| 3.47±0.04 | M—S |
| 3.30±0.04 | W |
| 2.74±0.02 | W |
| 2.52±0.02 | W |

These values were determined by standard techniques. The radiation was the K-alpha doublet of copper and a diffractometer equipped with a scintillation counter and an associated computer were used. The peak heights, I, and the positions as a function of 2 theta, where theta is the Bragg angle, were determined using algorithms on the computer associated with the spectrometer. From these, the relative intensities, 100 $I/I_o$, where $I_o$ is the intensity of the strongest line or peak, and d (obs.) the interplanar spacing in angstroms (A), corresponding to the recorded lines, were determined. In Table I, the relative intensities are given in terms of the symbols vs=very strong, s=strong, m=medium, and w=weak. It should be understood that this X-ray diffraction pattern is characteristic of all the species of ZSM-22 zeolite compositions. Ion exchange of the alkali or alkaline earth metal cations with other ions results in a zeolite which reveals substantially the same X-ray diffraction pattern as that of Table I with some minor shifts in interplanar spacing and variations in relative intensity. Other minor variations can occur, depending on the silica to alumina ratio of the particular sample, as well as its degree of thermal treatment.

The ZSM-22 zeolite freely sorbs normal hexane and has a pore dimension greater than about 4 Angstroms. In addition, the structure of the zeolite must provide constrained access to larger molecules. It is sometimes possible to judge from a known crystal structure whether such constrained access exists. For example, if the only pore windows in a crystal are formed by 8-membered rings of silicon and aluminum atoms, then access by molecules of larger cross-section than normal hexane is excluded and the zeolite is not of the desired type. Windows of 10-membered rings are preferred, although, in some instances, excessive puckering or pore blockage may render these zeolites ineffective. Twelve-membered rings do not generally appear to offer sufficient constraint to produce the advantageous hydrocarbon conversions, although puckered structures exist such as TMA offretite which is a known effective zeolite. Also, such twelve-membered structures can be conceived that may be operative due to pore blockage or other causes.

Rather than attempt to judge from crystal structure whether or not a zeolite possesses the necessary constrained access, a simple determination of the "constraint index" may be made. The meaning of constraint index and its method of determination are described in, for example, U.S. Patent No. 3905915. ZSM-22 has a constraint index of 2.6 at 800°F (427°C).

Preliminary data indicates that the ZSM-22 zeolite has an orthorhombic noncentral structure consisting substantially of 5 and 6-member rings which form a substantially unidirectional 10-ring channel system. Four member rings appear to be completely absent from the structure, which may explain, at least to some extent, the relatively high thermal stability of ZSM-22. (A sample of ZSM-22 was found to be thermally stable after heating at 550°C in air for 20 hours, and substantially steam stable, after the treatment at 920°F (493°C) for 5 hours in 1 atm saturated steam).

The as-synthesized ZSM-22 zeolite may be conveniently converted into the hydrogen, the univalent or multivalent cationic forms by base exchanging the zeolite to remove the alkali cations by such ions as hydrogen (from acids), ammonium, alkylammonium and arylammonium including $RNH_3$, $R_3NH^+$, $R_2NH_2^+$ and $R_4N^+$ where R is alkyl or aryl, provided that steric hindrance does not prevent the cations from entering

the cage and cavity structure of the ZSM-22 type crystalline zeolite. The hydrogen form of the zeolite is prepared, for example, by base exchanging the alkali form with, e.g., ammonium chloride or hydroxide, whereby the ammonium ion is substituted for the sodium ion. The composition is then calcined, at a temperature of, e.g., 1000°F (about 540°C), causing evolution of ammonia and retention of the hydrogen proton in the composition.

Zeolite ZSM-23 is described in U.S. Patents 4,076,842 and 4,104,151.

Platinum alone is preferably used as the hydrogenation/dehydrogenation component, although palladium, nickel, gold, zinc, gallium and rhenium or mixtures thereof may also be used. The amount of metal in elemental form will normally be in the range of 0.05 to 1 weight %, preferably 0.1 to 0.6 weight %, of the finished catalyst.

The finished catalyst preferably also includes a support preferably of alumina, although amorphous supports, such as silica, can also be employed.

The hydrogenation/dehydrogenation metal is preferably deposited at least on the zeolite, conveniently by ion exchange or impregnation techniques or by incorporation during synthesis of the zeolite. An extrudate of the zeolite and the alumina support can also be exchanged or impregnated with metal. Following deposition of the metal, it can be fixed in place by treatment with hydrogen sulfide, reduction to elemental metallic form or oxidation by calcination. The composition of the catalyst may be varied, but broadly may be given as within the following limits. All values are expressed in percentages by weight.

| | Broad | Preferred |
|---|---|---|
| Metal in elemental form | 0.1—1 | .1—.6 |
| Alumina support | 5—97 | 30—95 |
| ZSM-22 or ZSM-23 Zeolite | 3—80 | 5—70 |
| Additional binder or diluent | 0—20 | 0 |

The catalyst is preferably used in a fixed bed. The feedstream to the process ordinarily will comprise a stream of a mixture of xylenes and ethylbenzene with traces of other hydrocarbons such as paraffins and other nonaromatics. Ordinarily, the catalyst will be contacted with the feedstream at a weight hourly space velocity of 0.5 to 15, preferably 1—4, based on the catalyst, operating pressures will range from between 0 to 1000 psig (101 and 6996 kPa) and preferably between 100 and 500 psig (791 and 3549 kPa). Temperatures will be between 600 and 900°F (316 and 482°C), preferably between 750 and 850°F (399 and 454°C).

Examples

Example 1

A solution was prepared by mixing one part (by wt) aluminum sulfate, 4.5 parts potassium hydroxide (86% by wt) and 50 parts water. This solution was added to an autoclave. Another solution was prepared by mixing 27.6 parts colloidal silica (30% by wt) and 36 parts water and then this mixture was added to the autoclave. Six parts of diethylamine hydrochloride was then added and the combined solution was agitated vigorously for approximately one-half hour. The autoclave was heated to 330°F (166°C) with constant stirring and maintained for 72 hours at this temperature. The resultant crystalline material was then filtered and washed on a Buchner funnel and then dried overnight at 250°F (121°C). The X-ray diffraction analysis indicated that this material was ZSM-22. Chemical analysis gave a silica to alumina molar ratio of 82. This zeolite was mixed with alpha alumina to make a mixture of 65 parts by weight of zeolite and 35 parts by weight of alumina. Sufficient water was added to the mixture so that the resulting catalyst could be extruded into 1/16" pellets. These extrudate was then calcined in a nitrogen atmosphere at 1000°F (538°C). Subsequently the calcined extrudate was contacted with a 1.0 N ammonium nitrate solution and then calcined in air at 1000°F (538°C). The catalyst when tested had an alpha value of 57. The extrudate was then contacted with an aqueous solution of chloroplatinic acid to produce a finished catalyst having a platinum content of 0.6% by weight. The catalyst was then calcined at 900°F (482°C) for 3 hours. The alpha value of the resulting catalyst was 72. The catalyst composition was then contacted with a feedstream containing a mixture of toluene, ethylbenzene, para- meta- and orthoxylene in the percentages shown in Table II. The results of these tests are shown in Run Nos. 1 through 5 in Table II.

Comparative Example 1

In a similar test the Pt/mordenite catalyst described in Example 10 of U.S. Patent 4,128,591 was tested. The feedstock had a composition of about 30 percent ethylbenzene and 70 percent metaxylene. The results from this test are also tabulated in Table II.

The catalytic properties of the catalyst described above are compared in Table II to the best catalyst set forth in U.S. Patent 4,128,591. The catalyst activity is measured by the ethylbenzene conversion and paraxylene equilibrium approach, while the selectivity is best measured by the $C_8$ ring retention. It is easily seen from Table II that the present catalyst was both more active and selective than the platinum-mordenite

catalyst even though the hydrogen to hydrocarbon ratio was lower (i.e., 3 vs. 8) and the ethylbenzene content was higher (i.e., 40 vs. 30%) in the Examples according to the invention. For example, at 800°F (427°C), the $C_8$ ring retention for the platinum HZSM-22 catalyst was greater than 90 percent with the paraxylene equilibrium approach greater than 94 percent, while that for the platinum mordenite composition was approximately 88 percent with the paraxylene equilibrium approach being 92 percent.

TABLE II
Catalytic properties of Pt/HZSM-22 for xylene isomerization

| Run No. | 1* | 2* | 3* | 4* | 5* | Comparative** |
|---|---|---|---|---|---|---|
| Catalyst: | | | Pt/HZSM-22 | | | Pt/Mordenite ($SiO_2Al_2O_3$=14.4/1) |
| Temp. °F (°C) | 800 (427) | 850 (454) | 800 (427) | 800 (427) | 775 (413) | 825 (441) |
| Pressure, psig (kPa) | | | 200 (1480) | | | |
| $H_2$/HC | | | 3 | | | 8 |
| WHSV | | 3.1 | | 1.6 | | 3 |
| Time on stream, hrs | 13 | 26 | 38 | 51 | 242 | — |
| EB Conversion | 30 | 32 | 30 | 37 | 35 | — |
| P-Xylene equl'm app % | 96 | 98 | 94 | 101 | 102 | 92 |
| Xylene gain, wt % | 1.5 | −1.0 | 1.5 | 1.5 | 2.5 | — |
| $C_8$ Ring retention, mol % | 98 | 91 | 96 | 94 | 95 | 88 |
| $C_9^+$ Aromatics, wt % | 1.0 | 1.2 | 1.0 | 1.2 | 0.9 | — |
| BZ/EB, Mol ratio | 0.3 | 0.6 | 0.4 | 0.3 | 0.2 | — |

* Xylene feed composition: Toluene 1.1%, ethylbenzene 38.2%, para-xylene 6.1%, meta-xylene 41.4% and ortho-xylene 13.2%.
** U.S. Patent 4,128,591—Example 10 xylene feed: about 30% ethylbenzene, 70% meta-xylene.

Example 2 and Comparative Example 2

An HZSM-23 catalyst, containing 65% zeolite and 35% alumina by weight, was steamed to an alpha value of about 10 and then contacted with an aqueous solution of chloroplatinic acid to produce a metals loading of 0.06% platinum. The catalyst was then calcined in air at 900°F for 3 hours.

A platinum HZSM-5 catalyst was made from HZSM-5 steamed to an alpha value of about 5, the platinum being deposited on the zeolite to a concentration of 0.05% by weight. Again the finished catalyst contained 65% metallized, steamed zeolite and 35% alumina, by weight.

Table III compares the results obtained when the resultant ZSM-23 and ZSM-5 catalysts and the ZSM-22 catalyst of Example 1 were used to isomerize mixed xylene/ethylbenzene feeds.

TABLE III

| Catalyst: | | 0.6% Pt/HZSM-22 | 0.05% Pt/HZSM-23 | | 0.05% Pt/HZSM-5 |
|---|---|---|---|---|---|
| Temperature—°F (°C) | | 800 (427) | 800 (427) | | 794 (423) |
| Pressure, psig (kPa) | | 200 (1480) | 200 (1480) | | 200 (1480) |
| WHSV | | 3.1 | 3.0 | | 5.8 |
| H2/HC Molar ratio | | 2.6 | 2.8 | | 2.9 |
| Time on stream, hrs | | 13.0 | 20.0 | | 11.5 |
| EB Conversion, wt % | | 30.3 | 46.7 | | 62.6 |
| Xylene gain, wt %* | | 1.5 | 1.6 | | 1.3 |
| P-Xylene, equilibrium % | | 95.8 | 103.8 | | 104.0 |
| Prod dist, wt % | Feed | | | | |
| C1—C4 N.A. | | 1.5 | 2.9 | | 5.7 |
| C5—C7 N.A. | | 3.1 | 4.9 | | 1.5 |
| C8 Naphthene | | 1.3 | 1.4 | | 0.6 |
| C9 Naphthene | | | | | |
| Benzene | 0.0 | 2.2 | 5.2 | | 12.7 |
| Toluene | 0.7 | 2.7 | 1.4 | 1.1 | 2.0 |
| EB | 38.1 | 26.4 | 20.1 | 40.0 | 14.5 |
| P-Xylene | 6.1 | 14.2 | 14.8 | 6.1 | 14.5 |
| M-Xylene | 40.7 | 34.1 | 32.3 | 40.1 | 31.3 |
| O-Xylene | 14.5 | 13.7 | 14.8 | 13.7 | 14.5 |
| C9+ Arom | | 1.0 | 2.3 | | 2.6 |
| Total | 100.0 | 100.0 | 100.0 | 100.0 | 100.0 |

Comparative Example 3

HZSM-23 in a 65/35 weight ratio mixture with alumina was ion exchanged with platinum using an aqueous solution of $[Pt(NH_3)_4]Cl_2$ to achieve a platinum loading of 0.1% wt. After drying and calcining, the resultant Pt ZSM-23 was steamed to an alpha value of about 40.

The isomerization activity of the catalyst was then measured using the following feed and conditions:

| Feed (wt %) | Conditions |
|---|---|
| Toluene 1.5 | 135 psig (1320 kPa) |
| EB 8.0 | 800°F (427°C) |
| Para-xylene 9.0 | WHSV 10 $hr^{-1}$ |
| Meta-xylene 61 | $H_2$/HC 2 |
| Ortho-xylene 20.5 | |

and the following results were obtained:

| | |
|---|---|
| EB Conversion (wt %) | 0.18 |
| Para-xylene approach to equilibrium | 102.4 |
| Xylene gain | −0.18 |

Thus, in this comparative example, there was a net loss of xylene whereas in Example 2, where the ZSM-23 was steamed prior to platinum deposition, there was a net gain of xylene.

Example 3

In this Example, the effect of varying the isomerization temperature was investigated using the Pt ZSM-22 of Example 1. As will be seen from the results shown in Table IV, the isomerization temperature should preferably be maintained below 850°C.

Example 4

In this Example, the effect of varying the space velocity was monitored again using the Pt ZSM-22 of Example 1. As will be seen from the results shown in Table V, although the effects are temperature dependent, in general the space velocity is preferably maintained below 4.

TABLE IV
Xylene isomerization: effect of temperature

| | | | | | |
|---|---|---|---|---|---|
| Catalyst: | | 0.6% Pt/HZSM-22 | | Pressure 200 psig (1480 kPa) | |
| Temperature °F (°C) | | 775 (413) | 800 (427) | 850 (454) | 900 (482) |
| WHSV | | 2.9 | 3.1 | 3.0 | 3.2 |
| H2/HC Molar ratio | | 2.8 | 2.6 | 2.7 | 2.5 |
| Time on stream, hrs | | 254.0 | 13.0 | 26.0 | 32.0 |
| EB Conversion, wt % | | 26.1 | 30.3 | 32.3 | 46.6 |
| Xylene gain, wt %* | | 0.8 | 1.5 | −1.0 | −2.8 |
| Arom ring loss, M% | | 6.6 | 4.5 | 2.7 | 0.5 |
| P-Xylene, equilibrium % | | 91.6 | 95.8 | 98.1 | 100.5 |
| Prod dist, wt % | Feed | | | | |
| C1—C4 N.A. | | 1.0 | 1.5 | 2.5 | 3.8 |
| C5—C7 N.A. | | 3.0 | 3.1 | 1.9 | 1.1 |
| C8 Naphthene | | 3.7 | 1.3 | 0.6 | 0.0 |
| C9 Naphthene | | | | | |
| Benzene | 0.0 | 1.3 | 2.2 | 5.0 | 10.4 |
| Toluene | 0.7 | 1.2 | 2.7 | 2.8 | 3.8 |
| EB | 38.1 | 27.9 | 26.4 | 25.7 | 20.2 |
| P-Xylene | 6.1 | 13.7 | 14.2 | 14.0 | 13.8 |
| M-Xylene | 40.7 | 34.4 | 34.1 | 32.7 | 31.4 |
| O-Xylene | 14.5 | 13.2 | 13.7 | 13.8 | 14.1 |
| C9+ Arom | | 0.6 | 1.0 | 1.2 | 1.3 |
| Total | 100.0 | 100.0 | 100.0 | 100.0 | 100.0 |

TABLE V
Xylene isomerization: Effect of space velocity

| Catalyst: | | 0.6% Pt/HZSM-22 | | Pressure 200 psig (1480 kPa) | | |
|---|---|---|---|---|---|---|
| Temperature °F (°C) | | 800 (427) | 800 (427) | 800 (427) | 775 (413) | 775 (413) |
| WHSV | | 1.6 | 3.0 | 4.2 | 1.7 | 2.9 |
| H2/HC Molar ratio | | 2.6 | 2.7 | 3.0 | 2.5 | 2.8 |
| Time on stream, hrs | | 51.0 | 38.0 | 63.5 | 242.0 | 254.0 |
| EB Conversion, wt % | | 37.4 | 30.1 | 23.5 | 34.6 | 26.1 |
| Xylene gain, wt %* | | 1.5 | 1.5 | −1.7 | 2.5 | 0.8 |
| P-Xylene, equilibrium % | | 100.9 | 94.3 | 87.0 | 102.2 | 91.6 |
| Prod dist, wt % | Feed | | | | | |
| C1—C4 N.A. | | 3.0 | 1.5 | 1.3 | 1.9 | 1.0 |
| C5—C7 N.A. | | 3.8 | 2.6 | 2.3 | 4.2 | 3.0 |
| C8 Naphthene | | 1.0 | 1.2 | 1.3 | 2.3 | 3.7 |
| C9 Naphthene | | | | | | |
| Benzene | 0.0 | 3.2 | 3.0 | 1.9 | 1.7 | 1.3 |
| Toluene | 0.7 | 2.1 | 2.2 | 1.8 | 1.9 | 1.2 |
| EB | 38.1 | 23.7 | 26.5 | 29.0 | 24.7 | 27.9 |
| P-Xylene | 6.1 | 14.6 | 14.0 | 13.0 | 14.8 | 13.7 |
| M-Xylene | 40.7 | 33.2 | 34.1 | 33.9 | 33.7 | 34.4 |
| O-Xylene | 14.5 | 14.1 | 13.8 | 13.0 | 13.8 | 13.2 |
| C9+ Arom | | 1.2 | 1.0 | 2.5 | 0.9 | 0.6 |
| Total | 100.0 | 100.0 | 100.0 | 100.0 | 100.0 | 100.0 |

**Claims**

1. A proces for isomerizing ethylbenzene selectively to produce para xylene comprising passing ethyl benzene and hydrogen under isomerizing conditions over a catalyst comprising:
(a) ZSM 22- or ZSM-23 zeolite, and
(b) a hydrogenation/dehydrogenation metal, wherein the hydrogenation/dehydrogenation metal is incorporated in the catalyst after any steaming of the zeolite.
2. The process of Claim 1 wherein said metal is selected from the group consisting of platinum, palladium, nickel, gold, zinc or gallium or combinations of two or more thereof.
3. The process of Claims 1 or 2 wherein the catalyst also comprises a support.
4. The process of Claim 3 wherein the support comprises alumina.
5. The process of Claims 3 or 4 wherein the metal is incorporated at least on the zeolite.
6. The process of any preceding claim wherein the temperature is between 750—850°F (399—454°C).
7. The process of any preceding claims wherein the WHSV is between 1—4.

**Patentansprüche**

1. Verfahren zur selektiven Isomerisierung von Ethylbenzol zur Herstellung von p-Xylol, bei dem Ethylbenzol und Wasserstoff bei Isomerisierungsbedingungen über einen Katalysator geleitet werden, welcher umfaßt:

(a) einen Zeolith ZSM-22 oder ZSM-23 und

(b) ein Hydrierungs-/Dehydrierungsmetall, wobei das Hydrierungs-/Dehydrierungsmetall nach irgendeiner Dampfbehandlung des Zeolith in den Katalysator eingearbeitet wurde.

2. Verfahren nach Anspruch 1, worin das Metall aus der Gruppe ausgewählt ist, die aus Platin, Palladium, Nickel, Gold, Zink oder Gallium oder Kombinationen von zwei oder mehreren davon besteht.

3. Verfahren nach Anspruch 1 oder 2, worin dieser Katalysator ebenfalls ein Trägermaterial umfaßt.

4. Verfahren nach Anspruch 3, worin dieses Trägermaterial Aluminiumoxid umfaßt.

5. Verfahren nach Anspruch 3 oder 4, worin das Metall zumindest auf den Zeolith eingearbeitet ist.

6. Verfahren nach einem der vorstehenden Ansprüche, worin die Temperatur zwischen 750—850°F (399 bis 454°C) beträgt.

7. Verfahren nach einem der vorstehenden Ansprüche, worin die stündliche Gewichts-Raum-Geschwindigkeit zwischen 1 und 4 beträgt.

**Revendications**

1. Un procédé d'isomérisation sélective d'éthylbenzène en paraxylène consistant à faire passer de l'éthylbenzène et de l'hydrogène dans des conditions d'isomérisation sur un catalyseur comprenant:

(a) une zéolite ZSM-22 ou ZSM-23; et

(b) un métal d'hydrogénation/déshydrogénation, dans lequel le métal d'hydrogénation/déshydrogénation est incorporé dans le catalyseur après traitement de la zéolite à la vapeur.

2. Le procédé selon la revendication 1, dans lequel ce métal est choisi dans le groupe constitué par: platine, palladium, nickel, or, zinc ou gallium ou des combinaisons de deux ou de plus de deux de ces métaux.

3. Le procédé selon la revendication 1 ou 2, dans lequel le catalyseur comprend également un support.

4. Le procédé selon la revendication 3, dans lequel le support comprend de l'alumine.

5. Le procédé selon la revendication 3 ou 4, dans lequel le métal est au moins incorporé à la zéolite.

6. Le procédé selon l'une quelconque des revendications précédentes, dans lequel la température est comprise entre 399 et 454°C (750—850°F).

7. Le procédé selon l'une quelconque des revendications précédentes, dans lequel la VSHP est comprise entre 1 et 4.